(19) Europäisches Patentamt
European Patent Office
Office européen des brevets

(11) **EP 1 451 586 B1**

(12) **EUROPÄISCHE PATENTSCHRIFT**

(45) Veröffentlichungstag und Bekanntmachung des Hinweises auf die Patenterteilung:
**24.01.2007 Patentblatt 2007/04**

(21) Anmeldenummer: **03772302.0**

(22) Anmeldetag: **04.11.2003**

(51) Int Cl.:
*G01N 33/543* (2006.01) *G01N 27/02* (2006.01)

(86) Internationale Anmeldenummer:
**PCT/EP2003/012286**

(87) Internationale Veröffentlichungsnummer:
**WO 2004/042399 (21.05.2004 Gazette 2004/21)**

(54) **VERFAHREN UND VORRICHTUNG ZUR BESTIMMUNG DER KONZENTRATION VON IN EINER ZU UNTERSUCHENDEN PROBE ENTHALTENEN LIGANDEN**

METHOD AND DEVICE FOR DETERMINING A CONCENTRATION OF LIGANDS IN AN ANALYSED SAMPLE

PROCEDE ET DISPOSITIF POUR DETERMINER LA CONCENTRATION DE LIGANDS CONTENUS DANS UN ECHANTILLON A ANALYSER

(84) Benannte Vertragsstaaten:
**DE FR GB IT NL**

(30) Priorität: **05.11.2002 DE 10251757**

(43) Veröffentlichungstag der Anmeldung:
**01.09.2004 Patentblatt 2004/36**

(73) Patentinhaber:
- **Micronas Holding GmbH**
  **79108 Freiburg (DE)**
- **Klapproth, Holger, Dr.**
  **79108 Freiburg (DE)**

(72) Erfinder: **KLAPPROTH, Holger, Dr.**
**79108 Freiburg (DE)**

(74) Vertreter: **Huwer, Andreas**
**Grünwälderstrasse 10-14, Postfach 1305**
**79013 Freiburg i. Br. (DE)**

(56) Entgegenhaltungen:
**US-A1- 2002 051 975 US-B1- 6 197 503**
**US-B1- 6 203 981**

- **SCHWERDTLE R ET AL: "LABCHIPS UND MICROARRAYS FUER BIOTECHNOLOGISCHE ANWENDUNGEN LABORATORY CHIPS AND MICROARRAYS FOR BIOTECHNOLOGICAL APPLICATIONS" MEDIZINISCHE GENETIK, BERUFSVERBAND MEDIZINISCHE GENETIK, MUNCHEN,, DE, Bd. 11, Nr. 1, 1999, Seiten 29-32, XP009022868 ISSN: 0936-5931**

## Beschreibung

Die Erfindung betrifft ein Verfahren zur Bestimmung der Konzentration von in einer zu untersuchenden Probe enthaltenen Liganden, wobei auf wenigstens einem Träger Rezeptoren immobilisiert werden, die mit den Liganden eine spezifische Bindung eingehen können, wobei die Probe mit den Rezeptoren in Kontakt gebracht wird, wobei mindestens ein die Häufigkeit der Bindungen zwischen den Liganden und den Rezeptoren repräsentierender Messwert erfasst und anhand dieses Messwertes die Konzentration der Liganden in der Probe bestimmt wird. Außerdem bezieht sich die Erfindung eine Vorrichtung zur Bestimmung der Konzentration von in einer zu untersuchenden Probe enthaltenen Liganden, mit einem Träger, an dessen Oberfläche Rezeptoren immobilisiert sind, die beim Kontaktieren der Liganden mit diesen eine spezifische Bindung eingehen, und mit wenigstens einem Detektor zur Erfassung mindestens eines die Häufigkeit der Liganden-Rezeptor-Bindungen repräsentierenden Messwerts.

Ein derartiges Verfahren und eine derartige Vorrichtung sind aus Chris A Rowe-Taitt et al., Biosensors 8 Bioelectronics 15 (2000), Seite 579-589 bekannt. Die Vorrichtung weist als Träger einen CCD-Sensor auf, auf dessen Sensorzellen Antikörper immobilisiert sind. Die Antikörper dienen als Rezeptoren, die beim Kontaktieren des in der Probe enthaltenen Liganden diesen an sich binden, wodurch der Ligand auf dem CCD-Sensor in Form eines Rezeptor-Liganden-Komplexes immobilisiert wird. Die Antikörper sind so gewählt, dass sie für den Liganden spezifisch sind, d.h. andere in der Probe enthaltene Biomoleküle gehen beim Kontaktieren der Rezeptoren mit diesen keine Bindung ein. Ein Biomolekül kann Nukleinsäuren oder Derivate davon (DNA, RNA, PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen. Nachdem die Probe mit den Rezeptoren in Kontakt gebracht wurde und der Ligand an die Rezeptoren gebunden hat, wird eine Flüssigkeit auf den CCD-Sensor aufgebracht, die mit einem Fluoreszenzstoff markierte Antikörper enthält. Die Antikörper binden an die Liganden, wodurch diese mit dem Fluoreszenzstoff markiert werden. In einem weiteren Schritt wird die Sensoroberfläche mit den darauf immobilisierten Rezeptor-Liganden-Komplexen gewaschen, um mit dem Fluoreszenzstoff markierte Antikörper, die nicht an einen Rezeptor gebunden sind, von der Sensoroberfläche zu entfernen. Danach wird der Fluoreszenzstoff zur Anregung der Emission von Fluoreszenzstrahlung mit einer optischen Anregungsstrahlung bestrahlt. Die dabei freigesetzte Fluoreszenzstrahlung wird mit Hilfe der optischen Detektoren des CCD-Sensors gemessen. Sie stellt ein Maß für die Häufigkeit der Liganden-Rezeptor-Bindungen und somit für Konzentration des Liganden in der Probe dar.

Um eine quantitative Bestimmung der Konzentration des Liganden in der Probe zu ermöglichen, muss die Vorrichtung kalibriert werden. Bei einem aus der Praxis bekannten Kalibrierungsverfahren wird zu diesem Zweck zunächst eine Verdünnungsreihe hergestellt, die eine Anzahl von Kalibrierungsproben aufweist, welche den Liganden in unterschiedlichen, bekannten Konzentrationen enthält. Dies kann zum Beispiel in der Weise erfolgen, dass Liganden gewogen, anschließend mit einer bekannten Menge Flüssigkeit gemischt werden und dass von dem Gemisch unterschiedliche Verdünnungen hergestellt werden. Für die Durchführung der Kalibrierungsmessung wird eine der Anzahl der Kalibrierungsproben entsprechende Anzahl der eingangs erwähnten Vorrichtungen zur Bestimmung der Konzentration des Liganden bereit gestellt, wobei davon ausgegangen wird, dass diese Vorrichtungen untereinander und mit der vorstehend beschriebenen, für die eigentliche Konzentrationsmessung verwendeten Vorrichtung identisch sind. Mit Hilfe dieser Vorrichtungen werden die Kalibrierungsproben untersucht, wobei für die einzelnen Kalibrierungsproben jeweils ein Messwert für die Intensität der Fluoreszenzstrahlung erfasst wird. Auf diese Weise erhält man für jede Kalibrierungsprobe jeweils ein Wertepaar, bestehend aus der bekannten Konzentration des Liganden in der Kalibrierungsprobe und der für diese Konzentration gemessenen Intensität der Fluoreszenzstrahlung. Mit Hilfe dieser Wertepaare wird eine Kalibrierungskurve erstellt, für welche die Wertepaare Stützstellen bilden.

Die Kalibrierung hat den Nachteil, dass eine der Anzahl der Kalibrierungsproben entsprechende Anzahl Vorrichtungen benötigt wird, die anschließend nicht mehr für die Messung der zu untersuchenden Probe verwendet werden können. Die Kalibrierung der Vorrichtung ist deshalb aufwendig und teuer. Ungünstig ist außerdem, dass für die Kalibrierungskurve nur an den Stützstellen Messwerte vorliegen, und dass der Verlauf der Kalibrierungskurve zwischen den Stützstellen geschätzt werden muss, beispielsweise durch Interpolation. Schließlich ist aber auch problematisch, dass die Rezeptoren instabil sein können, was dazu führt, dass sich die Konzentration der auf dem Träger immobilisierten Rezeptoren zeitabhängig verändert. Die Kalibrierungsmessungen und die Messung an der zu untersuchenden Probe müssen deshalb möglichst zeitgleich erfolgen, wodurch zusätzlicher Aufwand entsteht.

Aus DE 197 36 641 A1 ist auch bereits ein Verfahren der eingangs genannten Art bekannt, bei dem zunächst eine Blindmessung durchgeführt wird, bei der Rezeptoren, die auf einem silanisierten Gals-Träger immobilisiert sind, mit einem Peroxidase-Tracer ohne Analyt in Kontakt gebracht werden. Bei der Blindmessung wird ein Messwert für die Anzahl der Bindungsereignisse bestimmt. Diese ist deutlich kleiner als die Anzahl der auf dem Träger immobilisierten Rezeptoren, da der Peroxidase-Tracer ein größeres Molekulargewicht aufweist als die Rezeptoren. Außerdem werden verschieden konzentrierte Kalibrierlösungen hergestellt, die den gesamten Messbereich umfassen. Mit diesen Kalibrierlösungen werden Kalibriermesswerte gemessen, aus denen mit dem Messwert der Blindmessung Kalibrierkurven erstellt werden. Danach wird die Vorrichtung regeneriert, indem die auf dem Träger immobilisierten Rezeptoren mit einer sauren Lösung in Kontakt gebracht werden, welche die Bindungen zwischen den Rezeptoren und dem Peroxidase-Tracer spalten. Danach werden die Rezeptoren mit der Probe in Kontakt gebracht und es werden Messwerte für die Häufigkeit der Bindungen zwischen den Liganden und den Rezeptoren ermittelt. Mit Hilfe dieser Messwerte und den Kalibrierkurven wird die Konzentration der Liganden in der Probe bestimmt. Das Verfahren hat den Nachteil, dass die Rezeptoren durch den Kontakt mit der sauren Lösung geschädigt werden können, wodurch Messfehler auftreten können. Ungünstig ist außerdem, dass das Verfahren relativ zeitaufwändig ist, weil bei der Kalibration abgewartet werden muss, bis sich die Bindungen zwischen den Rezeptoren und dem Peroxidase-Tracer ausgebildet haben und außerdem die Vorrichtung regeneriert werden muss.

Es besteht deshalb die Aufgabe, ein Verfahren und eine Vorrichtung der eingangs genannten Art zu schaffen, die es ermöglichen, auf einfache Weise die Konzentration eines in einer zu untersuchenden Probe enthaltenen Liganden zu bestimmen.

Diese Aufgabe wird bezüglich des Verfahrens dadurch gelöst, dass wenigstens ein Messwert für die Flächenbelegung der Oberfläche des Trägers mit den Rezeptoren ermittelt wird, und dass die Konzentration der Liganden mit Hilfe der Messwerte für die Flächenbelegung und die Häufigkeit der Bindungen bestimmt wird.

Bezüglich der Vorrichtung wird die vorstehend genannte Aufgabe dadurch gelöst, dass die Vorrichtung mindestens einen Flächenbelegungssensor zur Messung der Flächenbelegung der Oberfläche des Trägers mit den Rezeptoren aufweist, und dass der Flächenbelegungssensor und der Detektor zur Bestimmung der Konzentration des Liganden in der Probe mit einer Auswerteeinrichtung verbunden sind.

In vorteilhafter Weise ist es dadurch möglich, in entsprechender Anwendung des Massenwirkungsgesetzes die Ligandenkonzentration in der Probe auf einfache Weise wie folgt zu berechnen:

$$Ligandenkonzentration = K\,\frac{M}{F}.$$

Dabei bedeutet M der Messwert für die Häufigkeit der Bindungen zwischen den Liganden und den auf dem Träger immobilisierten Rezeptoren oder anders ausgedrückt für die Konzentration der auf dem Träger immobilisierten Rezeptor-Liganden-Komplexe, F der Messwert für die Flächenbelegung der Oberfläche des Trägers mit den Rezeptoren und K eine Bindungskonstante, die beispielsweise experimentell ermittelt werden kann. Dabei wird unter einer Flächenbelegung die Anzahl der Rezeptoren verstanden, die pro Oberflächeneinheit des Trägers auf diesem immobilisiert sind. Die Messwerterfassung für die Flächenbelegung der Oberfläche des Trägers mit den Rezeptoren erfolgt vorzugsweise unmittelbar bevor die zu untersuchende Probe mit den Rezeptoren in Kontakt gebracht wird. Somit kann auch bei instabilen Rezeptoren die Konzentration der Liganden in der Probe mit großer Genauigkeit anhand der zum Zeitpunkt der Erfassung des Messwerts oder der Messwerte für die Häufigkeit der Bindungen zwischen den Liganden und den Rezeptoren jeweils tatsächlich auf dem Träger vorhandenen Flächenbelegung bestimmt werden. Das erfindungsgemäße Verfahren und die erfindungsgemäße Vorrichtung ermöglichen es also, ohne ein aufwendiges und umständliches Anfertigen und Untersuchen einer Verdünnungsreihe, die Konzentration der Liganden in der Probe mit großer Präzision zu messen. Im Unterschied zu einer Blindwertmessung wird bei der Messung der Flächenbelegung nicht die Anzahl der Bindungsereignisse pro Fläche sondern die Anzahl der Rezeptoren pro Fläche gemessen. Es wird als eine Messgröße für die Sensitivität der Vorrichtung ermittelt. Die Flächenbelegung ist vor allem für die Dynamik der Messung von Bedeutung. Es hat sich nämlich gezeigt, dass mit zunehmender Flächenbelegung, d.h. mit zunehmender Dichte der Rezeptoren, die Wahrscheinlichkeit, dass ein Ligand an einen Rezeptor bindet, ansteigt. Dies hängt damit zusammen, dass das Bindungsereignis nur dann stattfinden kann, wenn der Ligand in die unmittelbare Nähe eines Rezeptors gelangt

Bei einer zweckmäßigen Ausgestaltung des Verfahren sind die Rezeptoren während der Messung der Flächenbelegung nicht an Liganden gebunden, und die Probe wird erst mit den Rezeptoren in Kontakt gebracht, nachdem die Flächenbelegung der Oberfläche des Trägers mit den Rezeptoren ermittelt wurde. Die Flächenbelegung kann dadurch mit großer Präzision bestimmt werden.

Bei einer vorteilhaften Ausführungsform der Erfindung ist der mindestens eine Flächenbelegungssensor ein Impedanzsensor. Nach dem Immobilisieren -der Rezeptoren auf der Oberfläche des Trägers wird zunächst zur Bestimmung des Messwerts für die Flächenbelegung die elektrische Impedanz an der Oberfläche des Trägers gemessen. Danach wird die Probe mit den Rezeptoren in Kontakt gebracht und es wird der mindestens eine Messwert für die Häufigkeit der Bindungen bestimmt. Der Impedanzsensor kann insbesondere als Kondensator ausgebildet sein, zwischen dessen Elektroden ein elektromagnetisches Wechselfeld anliegt. Die Rezeptoren wirken dabei als Dielektrikum, dessen Anwesenheit die elektrische Kapazität des Kondensators beeinflusst. Der Kondensator kann Teil eines Schwingkreises sein, dessen Resonanz- oder Schwingungsfrequenz zur Bestimmung der Flächenbelegung des Trägers mit Hilfe einer geeigneten Messeinrichtung erfasst wird.

Bei einer bevorzugten Ausführungsform der Erfindung ist der mindestens eine Flächenbelegungssensor ein Feldeffekttransistor, wobei der Träger vorzugsweise ein Halbleitersubstrat aufweist, in das der Feldeffekttransistor integriert ist. Der Träger ist also als Halbleiterchip ausgebildet, in den der mindestens eine Flächenbelegungssensor und gegebenenfalls der wenigstens eine Detektor zur Erfassung des die Häufigkeit der Liganden-Rezeptor-Bindungen repräsentierenden Messwerts kostengünstig integriert sei kann. Nach dem Immobilisieren der Rezeptoren auf der Oberfläche des Trägers wird zunächst zur Bestimmung des Messwerts für die Flächenbelegung die elektrische Feldstärke an der Oberfläche des Trägers gemessen. Danach wird die Probe mit den Rezeptoren in Kontakt gebracht und es wird der mindestens eine Messwert für die Häufigkeit der Bindungen bestimmt.

Bei einer zweckmäßigen Ausführungsform der Erfindung weist die Vorrichtung zur Anregung der Emission von Lumineszenzstrahlung in Abhängigkeit von der Bindung des Liganden an den Rezeptor mindestens eine optische Strahlungsquelle auf, in deren Abstrahlbereich die Rezeptoren angeordnet sind, wobei der wenigstens eine Detektor zum Detektieren der Lumineszenzstrahlung als Strahlungsempfänger ausgebildet ist, der vorzugsweise in das Halbleitersubstrat des Trägers integriert ist. Die an der Oberfläche des Trägers immobilisierten Rezeptor-Liganden-Komplexe können dann direkt oder indirekt über einen Antikörper oder dergleichen Biomolekül mit einem Lumineszenzstoff markiert sein. Es ist aber auch denkbar, dass der Ligand selbst durch die Strahlungsquelle zur Emission von Lumineszenzstrahlung angeregt wird. Schließlich kann die Konzentration oder die Häufigkeit der Rezeptor-Liganden-Komplexe aber auch dadurch bestimmt werden, dass während und/oder nach dem Inkontaktbringen der Liganden mit den Rezeptoren ein Kompetitor mit den Rezeptoren in Kontakt gebracht wird, der direkt oder indirekt mit einem Lumineszenzstoff markiert ist, dessen Lumineszenzstrahlung mit Hilfe des wenigstens einen Detektors gemessen wird.

Besonders vorteilhaft ist, wenn mehrere Detektoren matrixförmig in das Halbleitersubstrat integriert sind, und wenn mehrere Flächenbelegungssensoren über diese Matrix verteilt angeordnet sind, vorzugsweise zwischen den Detektoren. Dabei ist es insbesondere möglich, dass die Detektoren und die Flächenbelegungssensoren alternierend an der Oberfläche des Trägers angeordnet sind. Die Vorrichtung ermöglicht dann eine ortsaufgelöste Messung der Konzentration der Liganden in der Probe.

Nachfolgend ist ein Ausführungsbeispiel der Erfindung anhand der Zeichnung näher erläutert. Es zeigen zum Teil stärker schematisiert.

Fig. 1     einen Querschnitt durch eine Vorrichtung zur Bestimmung der Konzentration von in einer zu untersuchenden Probe enthaltenen Liganden, wobei die Vorrichtung eine Flusszelle aufweist,

Fig. 2     einen Teilquerschnitt durch einen Wandungsbereich der Flusszelle, der einen Detektor zur Detektion von an der Oberfläche des Wandungsbereichs befindlichen Liganden-Rezeptor-Komplexen und einen Fläche n-belegungssensor zur Messung der von Rezeptoren überdeckten Oberfläche des Wandungsbereichs aufweist, und

Fig. 3     eine Darstellung ähnlich Fig. 2, wobei der Rezeptor-Liganden-Komplex mit einem Lumineszenzstoff markiert ist, der mit Hilfe von Anregungs-Strahlung zur Abgabe von Lumineszenz-Strahlung angeregt wird.

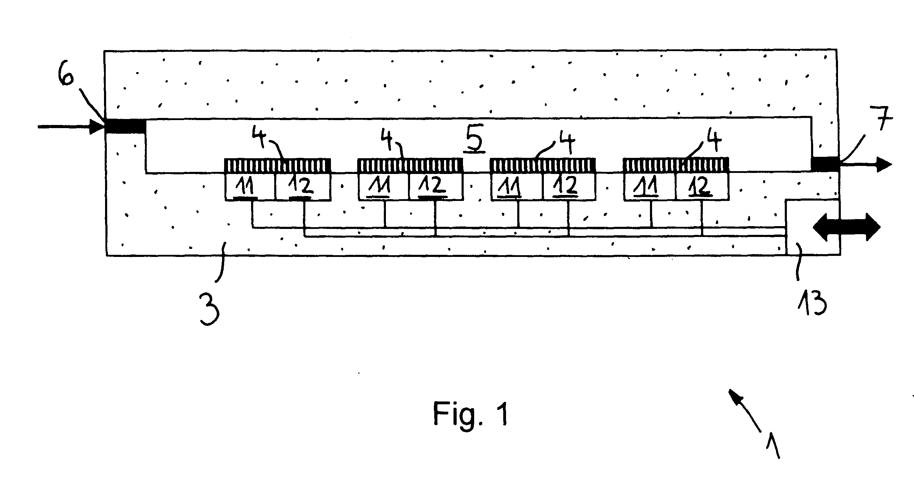

Eine im Ganzen mit 1 bezeichnete Vorrichtung zur Bestimmung der Konzentration von in einer zu untersuchenden Probe enthaltenen Liganden 2 hat einen Träger 3, an dessen Oberfläche biologische Antikörper als Rezeptoren 4 immobilisiert sind, die auf dem Träger 3 eine Monolage bilden. Die Immobilisierung der Rezeptoren 4 kann beispielsweise durch eine Silanisierung oder eine an der Oberfläche des Trägers 3 angeordnete Polyimidschicht erreicht werden, an welcher die Rezeptoren 4 anhaften. Die Rezeptoren 4 können auf den Träger 3 bzw. die darauf befindliche Polyimidschicht aufgedruckt sein. Wie in Fig. 1 erkennbar ist, sind an der Oberfläche des Trägers 3 mehre voneinander beabstandete Messpunkte mit Rezeptoren 4 gebildet.

Der Träger 3 ist durch einen Wandungsbereich einer Durchflussmesskammer gebildet, die eine Innenhöhlung 5 mit einer Einlassöffnung 6 und eine Auslassöffnung 7 für die Probe aufweist. Wie in Fig. 1 erkennbar ist, sind die Rezeptoren 4 an der der Innen höhlung 5 zugewandten Innenseite des Trägers 3 angeordnet. Zur Bestimmung der Konzentration der Liganden 2 in der Probe wird diese mit Hilfe einer Fördereinrichtung durch die Einlassöffnung 6 in die Innenhöhlung 5 der Messkammer eingebracht. Dabei kommen die Liganden 2 mit den Rezeptoren 4 in Kontakt.

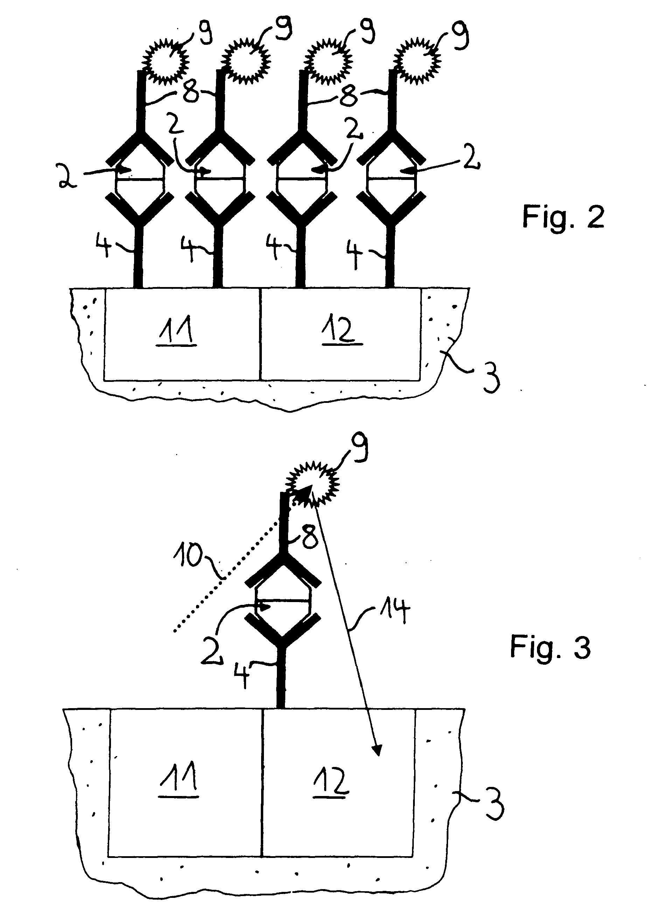

Die Liganden 2 weisen Eptiope auf, die beim Kontaktieren der Rezeptoren 4 mit diesen eine spezifische Bindung eingehen. Dabei werden die Liganden 2 in Form eines Rezeptor-Liganden-Komplexes auf dem Träger 3 immobilisiert, was in Fig. 2 und 3 schematisch dargestellt ist.

Mit den Liganden 2 werden sekundäre Antikörper 8 in Kontakt gebracht, die mit einem Lumineszenz-Stoff 9 markiert sind. Die sekundären Antikörper 8 binden an die Epitope der Liganden 2, wodurch die Rezeptor-Liganden-Komplexe mit dem Lumineszenz-Stoff 9 markiert werden. Es ist aber auch denkbar, dass die Rezeptor-Liganden-Komplexe beispielsweise auf Grund von Chemilumineszeriz selbst lumineszent sind. In diesem Fall kann das Markieren der Rezeptor-Liganden-Komplexe mit dem Lumineszenz-Stoff 9 gegebenenfalls entfallen.

In einem weiteren Schritt wird der Träger 3 zum Entfernen der nicht an einen Rezeptor 4 gebundenen Bestandteile der Probe gewaschen. Dies kann beispielsweise in der Weise geschehen, dass durch die Einlassöffnung 6 eine Spülflüssigkeit in die Innenhöhlung eingebracht wird, mit deren Hilfe die nicht auf dem Träger immobilisierten Bestandteile der Probe über die Auslassöffnung 7 aus der Innenhöhlung 5 herausgespült werden.

Danach werden die weiterhin an die Rezeptoren 4 gebundenen Lumineszenz-Stoffe 8 mit Hilfe einer in der Zeichnung nicht näher dargestellten Strahlungsquelle mit einer Anregungsstrahlung 10 bestrahlt. Das Spektrum der Anregungsstrahlung 10 weist mindestens eine Anregungswellenlänge auf, bei welcher der Lumineszenz-Stoff 9 zur Abgabe von Lumineszenzstrahlung 14 angeregt wird.

Der Träger 3 hat ein Halbleitersubstrat, in das Detektoren 11 integriert sind, die zur Messung der Lumineszenzstrahlung 14 als optische Strahlungsempfänger ausgebildet sind, beispielsweise als Photodioden. Die Detektoren 8 sind matrixförmig in mehreren Reihen und Spalten angeordnet. Mit Hilfe der Detektoren 8 werden ortsaufgelöst Messwerte erfasst, die ein Maß für die Anzahl der Rezeptor-Liganden-Komplexe pro Fläche des Trägers 3 darstellen.

Zur Messung der Flächenbelegung der Oberfläche des Trägers 3 mit den Rezeptoren 4 sind in das Halbleitersubstrat des Trägers 3 Flächenbelegungssensoren 12 integriert. Die Flächenbelegungssensoren 12 sind als Feldeffekttransistoren (FET) ausgebildet, deren Gate zur Detektion der Rezeptoren 4 derart an der Oberfläche des Trägers 3 oder in einer obefflüchennahen Schicht des Trägers 3 angeordnet ist, dass die auf dem Träger immobilisierten Rezeptoren 4 das Gate elektrisch kontaktieren. In Fig. 1 ist erkennbar, dass die Flächenbelegungssensoren 12 und die Detektoren 8 einander abwechselnd nebeneinander angeordnet sind und dass für jeden durch einen Rezeptorbereich gebildeten Messpunkt jeweils wenigstens ein Flächenbelegungssensor 12 und mindestens ein Detektor 8 vorgesehen ist. Die Messung der Flächenbelegung der Trägeroberfläche mit den Rezeptoren 4 erfolgt vorzugsweise unmittelbar bevor die Probe mit den Rezeptoren 4 in Kontakt gebracht wird. Dabei wird davon ausgegangen, dass sich Flächenbelegung zwischen dieser Messung und der Bestimmung der Häufigkeit oder Konzentration der Rezeptor-Liganden-Komplexe durch Messung der Lumineszenzstrahlung 14 nicht oder nur unwesentlich verändert. Der Messwert für die Flächenbelegung stellt ein Maß für die Anzahl der Rezeptoren 4 pro Trägeroberfläche dar.

Zur Bestimmung der Konzentration der Liganden 2 in der Probe sind die Flächenbelegungssensoren 12 und die Detektoren 8 mit einer Auswerteeinrichtung 13 verbunden, die bei dem Ausführungsbeispiel nach Fig.1 in den Träger 3 integriert ist. Mit Hilfe der Auswerteeinrichtung 13 wird die Konzentration der Liganden 2 aus einem die Häufigkeit der Rezeptor-Liganden-Komplexe repräsentierenden Messwert M einem Messwert F für die Flächenbelegung der Oberfläche des Trägers 3 mit den Rezeptoren 4 und einer Bindungskonstanten K wie folgt bestimmt.

$$Ligandenkonzentration = K \frac{M}{F}.$$

Dabei ist es sogar möglich, die Konzentration der Liganden 2 für einzelne Messpunkte oder für Gruppen von mehreren Messpunkten getrennt zu ermitteln, so dass sich insgesamt eine ortsaufgelöste ein- oder sogar zweidimensionale Konzentrationsmessung ergibt.

Die Auswerteeinrichtung 13 weist eine in der Zeichnung nur schematisch dargestellte Schnittstelleneinrichtung zum Verbinden mit einer übergeordneten Anzeige- und/oder Auswerteeinheit auf, beispielsweise einem Mikrocomputer. Auf der Anzeige können die Messwerte für die Flächenbelegung, die Lumineszenzstrahlung 14 und/oder die daraus berechnete Konzentration der Liganden 2 graphisch dargestellt werden.

Bei dem Verfahren zur Bestimmung der Konzentration von in einer zu untersuchenden Probe enthaltenen Liganden 2 werden also auf einem Träger 3 Rezeptoren 4 immobilisiert, die mit den Liganden 2 eine spezifische Bindung eingehen können. Mit Hilfe mindestens eines Flächenbelegungssensors 12 wird wenigstens ein Messwert für die Flächenbelegung der Oberfläche des Trägers 3 mit den Rezeptoren 4 erfasst Danach wird die Probe mit den Rezeptoren 4 in Kontakt gebracht. Mittels wenigstens eines Detektors 11 wird mindestens ein die Häufigkeit der Bindungen zwischen den Liganden 2 und den Rezeptoren 4 repräsentierender Messwert erfasst. Anhand der Messwerte für die Flächenbelegung und die Häufigkeit der Liganden-Rezeptor-Bindungen wird die Konzentration der Liganden 2 in der Probe bestimmt.

**Patentansprüche**

1. Verfahren zur Bestimmung der Konzentration von in einer zu untersuchenden Probe enthaltenen Liganden (2), wobei auf wenigstens einem Träger (3) Rezeptoren (4) immobilisiert werden, die mit den Liganden (2) eine spezifische Bindung eingehen können, wobei die Probe mit den Rezeptoren (4) in Kontakt gebracht wird, wobei mindestens ein die Häufigkeit der Bindungen zwischen den Liganden (2) und den Rezeptoren (4) repräsentierender Messwert erfasst und anhand dieses Messwertes die Konzentration der Liganden (2) in der Probe bestimmt wird, **dadurch gekennzeichnet dass** wenigstens ein Messwert für die Flächenbelegung der Oberfläche des Trägers (3) mit den Rezeptoren (4) ermittelt wird, und dass die Konzentration der Liganden (2) mit Hilfe der Messwerte für die Flächenbelegung und die Häufigkeit der Bindungen bestimmt wird.

2. Verfahren nach Anspruch 1, **dadurch gekennzeichnet, dass** die Rezeptoren (4) während der Messung der Flächenbelegung nicht an Liganden (2) gebunden sind, und dass die Probe mit den Rezeptoren (4) in Kontakt gebracht wird, nachdem die Flächenbelegung der Oberfläche des Trägers (3) mit den Rezeptoren (4) ermittelt wurde.

3. Verfahren nach Anspruch 1 oder 2, **dadurch gekennzeichnet, dass** nach dem Immobilisieren der Rezeptoren (4) auf der Oberfläche des Trägers (3) zur Bestimmung des Messwerts für die Flächenbelegung die elektrische Impedanz an der Oberfläche des Trägers (3) gemessen wird, und dass danach die Probe mit den Rezeptoren (4) in Kontakt gebracht und der mindestens eine Messwert für die Häufigkeit der Bindungen bestimmt wird.

4. Verfahren nach einem der Ansprüche 1 bis 3, **dadurch gekennzeichnet, dass** nach dem Immobilisieren der Rezeptoren (4) auf der Oberfläche des Trägers (3) zur Bestimmung des Messwerts für die Flächenbelegung die elektrische Feldstärke an der Oberfläche des Trägers (3) gemessen wird, und dass danach die Probe mit den Rezeptoren (4) in Kontakt gebracht und der mindestens eine Messwert für die Häufigkeit der Bindungen bestimmt wird.

5. Vorrichtung (1) zur Bestimmung der Konzentration von in einer zu untersuchenden Probe enthaltenen Liganden (2), mit einem Träger (3), an dessen Oberfläche Rezeptoren (4) immobilisiert sind, die beim Kontaktieren der Liganden (2) mit diesen eine spezifische Bindung eingehen, und mit wenigstens einem Detektor (11) zur Erfassung mindestens eines die Häufigkeit der Liganden-Rezeptor-Bindungen repräsentierenden Messwerts, **dadurch gekennzeichnet**, da ss die Vorrichtung (1) mindestens einen Flächenbelegungssensor (12) zur Messung der Flächenbelegung der Oberfläche des Trägers (3) mit den Rezeptoren (4) aufweist, und dass der Flächenbelegungssensor (12) und der Detektor (11) zur Bestimmung der Konzentration des Liganden (2) in der Probe mit einer Auswerteeinrichtung (13) verbunden sind.

6. Vorrichtung (1) nach Anspruch 5, **dadurch gekennzeichnet, dass** der mindestens eine Flächenbelegungssensor (12) ein Impedanzsensor ist.

7. Vorrichtung (1) nach Anspruch 5 oder 6, **dadurch gekennzeichnet, dass** der mindestens eine Flächenbelegungssensor (12) ein Feldeffekttransistor ist, und dass der Träger (3) vorzugsweise ein Halbleitersubstrat aufweist, in das der Feldeffekttransistor integriert ist.

8. Vorrichtung (1) nach einem der Ansprüche 5 bis 7, **dadurch gekennzeichnet, dass** sie zur Anregung der Emission von Lumineszenzstrahlung (14) in Abhängigkeit von der Bindung des Liganden (2) an den Rezeptor (4) mindestens eine optische Strahlungsquelle aufweist, in deren Abstrahlbereich die Rezeptoren (4) angeordnet sind, und dass der wenigstens eine Detektor (11) zum Detektieren der Lumineszenzstrahlung (14) als Strahlungsempfänger ausgebildet ist, der vorzugsweise in das Halbleitersubstrat des Trägers (3) integriert ist.

9. Vorrichtung nach einem der Ansprüche 5 bis 8, **dadurch gekennzeichnet, dass** mehrere Detektoren (11) matrixförmig in das Halbleitersubstrat integriert sind, und dass mehrere Flächenbelegungssensoren (12) über diese Matrix verteilt angeordnet sind, vorzugsweise zwischen den Detektoren (11).

**Claims**

1. Method for the determination of the concentration of ligands (2) contained in a sample to be analysed, whereby receptors (4) that can enter into a specific bond with the ligands (2) are immobilized on at least one support (3), whereby the sample is brought into contact with the receptors (4), whereby at least one measurement value representing the frequency of the bonds between the ligands (2) and the receptors (4) is measured, and on the basis of this measurement value the concentration of the ligands (2) in the sample is determined, **characterized in that** at least one measurement value of the surface occupation of the surface of the support (3) with the receptors (4) is determined, and that the concentration of the ligands (2) is determined by means of the measurement values for the surface occupation and the frequency of the bonds.

2. Method according to Claim 1, **characterized in that** the receptors (4) are not bonded to ligands (2) during the measurement of the surface occupation, and that the sample is brought into contact with the receptors (4), after the surface occupation of the surface of the support (3) with the receptors (4) has been determined.

3. Method according to Claim 1 or 2, **characterized in that** after the immobilization of the receptors (4) on the surface of the support (3), for the determination of the measurement value for the surface occupation, the electrical impedance is measured on the surface of the support (3), and that then the sample is brought into contact with the receptors (4) and the at least one measurement value for the frequency of the bonds is determined.

4. Method according to any of Claims 1 to 3, **characterized in that** after the immobilization of the receptors (4) on the surface of the support (3) for the determination of the measurement value for the surface occupation, the electrical field strength on the surface of the support (3) is measured, and that then the sample is brought into contact with the receptors (4) and the at least one measurement value for the frequency of the bonds is determined.

5. Device (1) for the determination of the concentration of ligands (2) contained in a sample to be analysed, with a support (3) on the surface of which receptors (4) are immobilized which, when they come into contact with the ligands (2) can enter into a specific bond with the ligands, and with at least one detector (11) for the determination of at least one measurement value that represents the frequency of the ligand-receptor bonds, **characterized in that** the device (1) has at least one surface occupation sensor (12) for the measurement of the surface occupation of the surface of the support (3) with the receptors (4), and that the surface occupation sensor (12) and the detector (11) for the determination of the concentration of the ligand (2) in the sample are connected with an evaluation device (13).

6. Device (1) according to Claim 5, **characterized in that** the at least one surface occupation sensor (12) is an impedance sensor.

7. Device according to Claim 5 or 6, **characterized in that** the at least one surface occupation sensor (12) is a field effect transistor, and that the support (3) preferably has a semiconductor substrate, into which the field effect transistor is integrated.

8. Device (1) according to any of Claims 5 to 7, **characterized in that** it has at least one optical radiation source for the excitation of the emission of luminescent radiation (14) as a function of the bonding of the ligand (2) to the receptor (4), in the radiation range of which the receptors (4) are located, and that the at least one detector (11) is realized for the determination of the luminescent radiation (14) in the form of a radiation receiver, which is preferably integrated into the semiconductor substrate of the support (3).

9. Device according to any of Claims 5 to 8, **characterized in that** a plurality of detectors (11) are integrated in a matrix pattern into the semiconductor substrate, and that a plurality of surface occupation sensors (12) are distributed over this matrix, preferably between the detectors (11).

## Revendications

1. Procédé de détermination de la concentration de ligands (2) contenus dans un échantillon à examiner, dans lequel des récepteurs (4), qui peuvent donner lieu avec les ligands (2) à une liaison spécifique, sont immobilisés sur au moins un support (3), l'échantillon étant amené en contact avec les récepteurs (4), au moins une valeur de mesure représentant l'abondance des liaisons entre les ligands (2) et les récepteurs (4) étant détectée et la concentration des ligands (2) dans l'échantillon étant déterminée à l'aide de cette valeur de mesure, **caractérisé en ce qu'**au moins une valeur de mesure du recouvrement de la surface du support (3) par les récepteurs (4) est établie et **en ce que** la concentration des ligands (2) est déterminée à l'aide des valeurs de mesure du recouvrement de surface et de l'abondance des liaisons.

2. Procédé suivant la revendication 1, **caractérisé en ce que** les récepteurs (4) ne sont pas liés aux ligands (2) pendant la mesure du recouvrement de surface et **en ce que** l'échantillon est amené en contact avec les récepteurs (4) après que le recouvrement de la surface du support (3) par les récepteurs (4) a été établi.

3. Procédé suivant la revendication 1 ou 2, **caractérisé en ce que**, après l'immobilisation des récepteurs (4) sur la surface du support (3), on mesure, pour déterminer la valeur de mesure du recouvrement de surface, l'impédance électrique à la surface du support (3) et **en ce qu'**ensuite l'échantillon est amené en contact avec les récepteurs (4) et ladite au moins une valeur de mesure de l'abondance des liaisons est déterminée.

4. Procédé suivant l'une des revendications 1 à 3, **caractérisé en ce que**, après l'immobilisation des récepteurs (4) à la surface du support (3), on mesure, pour déterminer la valeur de mesure du recouvrement de surface, l'intensité de champ électrique à la surface du support (3) et **en ce qu'**ensuite l'échantillon est amené en contact avec les récepteurs (4) et ladite au moins une valeur de mesure de l'abondance des liaisons est déterminée.

5. Dispositif (1) pour déterminer la concentration de ligands (2) contenus dans un échantillon à examiner, comprenant un support (3) sur la surface duquel sont immobilisés des récepteurs (4) qui, lors d'une mise en contact des ligands (2) avec ceux-ci, donnent lieu à une liaison spécifique, et au moins un détecteur (11) pour détecter au moins une valeur de mesure représentant l'abondance des liaisons ligands-récepteurs, **caractérisé en ce que** le dispositif (1) présente au moins un capteur de recouvrement de surface (12) pour mesurer le recouvrement de la surface du support (3) par les récepteurs (4) et **en ce que** le capteur de recouvrement de surface (12) et le détecteur (11) sont, pour déterminer la concentration des ligands (2) dans l'échantillon, connectés à un dispositif d'évaluation (13).

6. Dispositif (1) suivant la revendication 5, **caractérisé en ce que** ledit au moins un capteur de recouvrement de surface (12) est un capteur d'impédance.

7. Dispositif (1) suivant la revendication 5 ou 6, **caractérisé en ce que** ledit au moins un capteur de recouvrement de surface (12) est un transistor à effet de champ et **en ce que** le support (3) présente de préférence un substrat à semi-conducteur dans lequel le transistor à effet de champ est intégré.

8. Dispositif (1) suivant l'une des revendications 5 à 7, **caractérisé en ce que**, pour l'excitation de l'émission d'un rayonnement luminescent (14) en fonction de la liaison du ligand (2) sur le récepteur (4), il présente au moins une source de rayonnement optique dans la zone de l'irradiation de laquelle les récepteurs (4) sont agencés et **en ce que** ledit au moins un détecteur (11) destiné à détecter le rayonnement luminescent (14) est réalisé sous la forme d'un capteur de rayonnement qui de préférence est intégré dans le substrat à semi-conducteur du support (3).

9. Dispositif suivant l'une des revendications 5 à 8, **caractérisé en ce que** plusieurs détecteurs (11) sont intégrés en forme de matrice dans le substrat à semi-conducteur et **en ce que** plusieurs capteurs de recouvrement de surface (12) sont agencés de manière répartie sur cette matrice, de préférence entre les détecteurs (11).

Fig. 1

EP 1 451 586 B1

Fig. 2

Fig. 3